# EUROPEAN PATENT SPECIFICATION

(11) **EP 0 644 197 B1**
(45) Date of publication and mention of the grant of the patent: **09.09.1998**
(21) Application number: 94201570.2
(22) Date of filing: 02.06.1994
(51) Int. Cl.: C07K 5/023, C07F 9/32, C07F 9/40, C07F 9/48, A61K 31/66, A61K 38/04

(54) **Peptidic phosphinyloxymethyl ketones as interleukin-1beta-converting enzyme inhibitors**
Peptid-Phosphinyloxymethyl-Ketonen als Inhibitoren von Interleukin-1 beta-konvertierenden Enzymen
Peptidyl-phosphinyloxyméthyl-cétones comme inhibiteurs d'enzyme convertissant l'interleukine-1 bêta

(30) Priority: 04.06.1993 US 73219
(43) Date of publication of application: 22.03.1995
(73) Proprietor: VERTEX PHARMACEUTICALS INCORPORATED, Cambridge, MA 02139-4242 (US)
(72) Inventor: Dolle, Roland E., c/o Sterling Winthrop Inc., New York, New York 10016 (US); Singh, Jasbir, c/o Sterling Winthrop Inc., New York, New York 10016 (US)
(74) Representative: Le Guen, Gérard

(56) References cited:
- WO-A-93/05071
- WO-A-93/09135

## Description

This invention relates to a series of novel amino acid, di- and polypeptide analogs which exhibit selective inhibition of interleukin-1β-converting enzyme, to compositions containing the novel amino acid analogs and methods for therapeutic utility. The interleukin-1β-converting enzyme inhibitors described in this invention comprise novel aspartic acid-derived phosphinyloxymethyl ketones which possess particular utility in the treatment of inflammatory and immune-based diseases and cancer.

Interleukin-1β protease (also known as interleukin-1β-converting enzyme or ICE) is the enzyme responsible for processing of the biologically inactive 31 kD precursor IL-1β to the biologically active 17 kD form (Kostura, M.J.; Tocci, M.J.; Limjuco, G.; Chin, J.; Cameron, P.; Hillman, A.G.; Chartrain, N.A.; Schmidt, J.A. Proc. Nat. Acad. Sci., 1989, 86, 5227-5231 and Black, R.A.; Kronheim, S.R.; Sleath, P.R. FEBS Let., 1989, 247, 386-391). In addition to acting as one of the body's early responses to injury and infection, IL-1β has also been proposed to act as a mediator of a wide variety of diseases, including rheumatoid arthritis, osteoarthritis, inflammatory bowel disease, sepsis, and acute and chronic myelogenous leukemia (Dinarello, C.A.; Wolff, S.M., New Engl. J. Med., 1993, 328, 106). The naturally occurring IL-1β receptor antagonist has been used to demonstrate the intermediacy of IL-1β in a number of human diseases and animal models (Hannum, C.H.; Wilcox, C.J.; Arend, W.P.; Joslin, G.G.; Dripps, D.J.; Heimdal, P.L.; Armes, L.G.; Sommer, A.; Eisenberg, S.P.; Thompson, R.C., Nature, 1990, 343, 336-340; Eisenberg, S.P.; Evans, R.J.; Arend, W.P.; Verderber, E.; Brewer, M.T.; Hannum, C.H.; Thompson, R.C., Nature 1990, 343, 341-346; Ohlsson, K.; Bjork, P.; Bergenfeldt, M.; Hageman, R.; Thompson, R.C., Nature, 1990, 348, 550-552; and Wakabayashi, G., GASEB, 1991, 338-343). The specific role of IL-1β in inflammation and immunomodulation is supported by the recent observation that the cowpox virus employs an inhibitor of ICE to suppress the inflammatory response of its host (Ray, C.A. et al, Cell, 1992, 69, 597-604).

The present invention also relates to the modulation of processing of IL-1β for the treatment of rheumatoid arthritis Levels of IL-1β are known to be elevated in the synovial fluid of patients with the disease. Additionally, IL-1β stimulates the synthesis of enzymes believed to be involved in inflammation, such as collagenase and PLA_{2,} and produces joint destruction which is very similar to rheumatoid arthritis following intra-articular injection in animals.

A limited number of peptidyl methyl ketone analogs constitute a well-known class of compounds having cysteine protease (papain, cathepsin B) inhibitory activity. These peptidyl methyl ketone analogs have been reviewed by D. Rich in Chapter 4 of "Proteinase Inhibitors", Barrett, A.J. and Salvensen, G., eds., Elsevier, 1986. More recently, α-aryloxy and α-arylacyloxy methyl ketones have also been described as inhibitors of cysteine protease (Krantz, A. et al, Biochemistry, 30, p. 4678-4687, 1991).

These peptide analogs, however, are essentially devoid of potency and selectivity in inhibiting ICE.

WO 93/05071 discloses oligopeptide inhibitors of interleukin-1β-converting enzyme of the formula: Z-Q₂-Asp-Q₁ in which
Z is an N-terminal blocking group;
Q₂ is an amino acid sequence;
Asp designates aspartic acid; and
Q₁ is an electronegative group.

As illustrative examples of such electronegative groups aldehyde, diazoalkyl ketone and haloalkyl ketone are mentioned.

WO 93/09 135 relates to peptides inhibiting interleukin-1β-release of the formula R-[A₁-A₂]n-A₃-A₄-X-A₅ in which X may represent

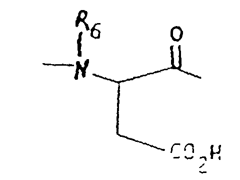

However according to this document A₅ does not include a phosphorus atom.

An effective therapy has yet to be developed for the treatment of IL-1β mediated inflammatory diseases. Consequently, there is a need for therapeutic agents effective in the treatment and prevention of these diseases.

According to the present invention, there is provided a compound of the formula (I) and a pharmaceutically acceptable salt thereof: 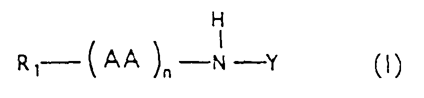 wherein:
n is 0 - 4;
Y is 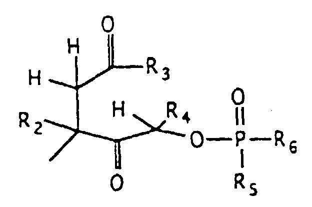
and when R₃ is OH, then Y can also be 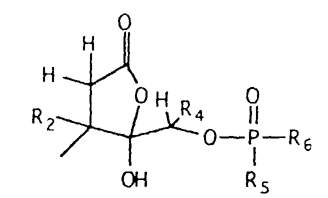
R₂ is H or deuterium;
R₃ is OH, OR₇, NR₇OR₈ or NR₇R₈;
where R₇ and R₈ are independently H, alkyl, cycloalkyl, aralkyl, heteroaralkyl, aryl or heteroaryl;
R₄ is H or lower alkyl;
R₅ and R₆ are optionally and independently selected from H, OH, alkyl, aryl, aralkyl, heteroaryl, heteroaralkyl, alkoxy, aroxy, heteroaroxy, aralkoxy, heteroaralkoxy, alkenyl, aralkenyl or heteroaralkenyl;

AA is independently selected from the group consisting of (a) and (b) where (a) is defined as an amino acid of formula II 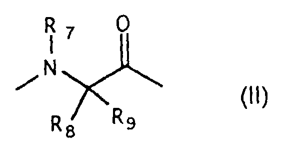
wherein R₇ and R₈ are defined as above and R₉ is (CR₆R₇)₀₋₆-R₁₀;
wherein R₁₀ is a radical optionally selected from R₁₁, where R₁₁ is described below; and
wherein group (b) is selected from the group consisting of: 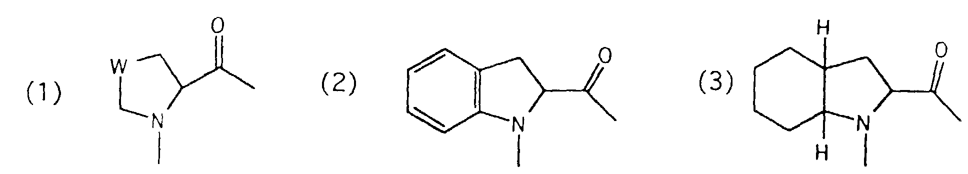 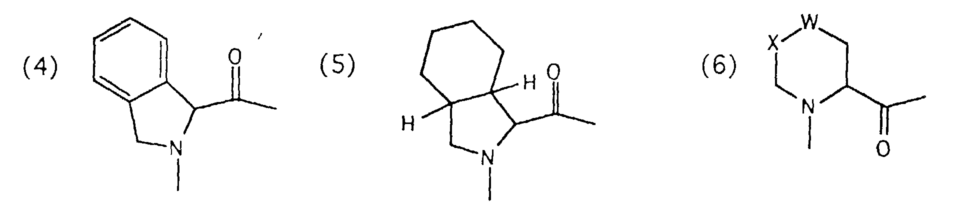 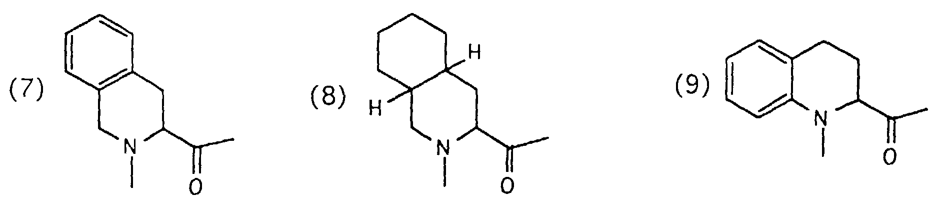 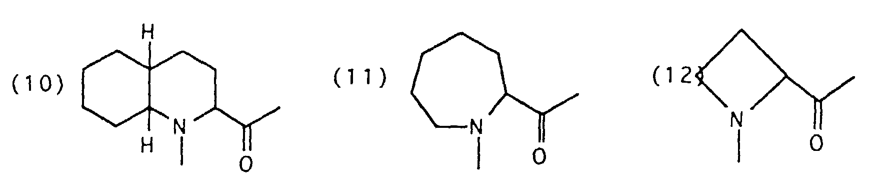 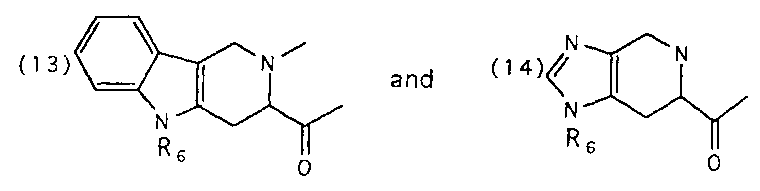
where W and X are optionally CH₂, O, S or NR₇;
R₁ is R₁₀-CO- or R₁₀SO₂-, where R₁₀ is defined previously;
R₁₁ is H, alkyl, alkenyl, aryl, heteroaryl, aralkyl, heteroaralkyl, aralkenyl, heteroaralkenyl, hydroxy, alkoxy, 2-(alkoxy)ethoxy, 2-(alkoxy)aminoethyl, 2-(alkoxy)-N-alkylaminoethyl, aralkoxy, heteroaralkoxy, alkylacyloxy, aralkylacyloxy, heteroaralkylacyloxy, aracyloxy, heteroaracyloxy, aryloxyalkylacyloxy, heteroaryloxyalkylacyloxy, alkylacyl, aralkylacyl, heteroaralkylacyl, alkylacylamino, aralkylacylamino, heteroaralkylacylamino, aracylamino, heleroaracylamino, aryloxyalkylacylamino, heteroaryloxyalkylacylamino, alkyloxyalkylacylamino, alkoxyacylamino, aralkoxyacylamino, heteroaralkoxyacylamino, aracyl, heteroaracyl, aryloxyalkylacyl, heteroaryloxyalkylacyl, halo, haloalkyl, guanidino, mono- and di-alkylguanidino, mono- and di-aralkylguanidino, mono- and di-heteroaralkylguanidino, alkylacylguanidino, aralkylacylguanidino, heteroaralkylguanidino, aracylguanidino, heteroarylguanidino, amidino, mono- and di-alkylamidino, mono- and diaralkylamidino, mono- and di-heteroaralkylamidino, amino, mono- and dialkylamino, mono- and diaralkylamino, mono- and di-heteroaralkylamino, carboxy, alkylcarboxy, carbalkoxy, carbaralkoxy, carbheteroaralkoxy, carbalkoxyalkenyl, carboxamido, mono- and dialkylcarboxamido, mono- and diarcarboxamido, mono and di-heteroarcarboxamido, mono- and di-aralkylcarboxamido, mono- and di-heteroaralkylcarboxamido, thio, alkylthio, arylthio, heteroarylthio, aralkylthio, heteroaralkylthio, sulfonamido, mono- and di-alkylsulfonamido, mono- and di-aralkylsulfonamido, mono- and di-heteroaralkylsulfonamido, morpholinosulfonamido, alkylsulfonyl, aralkylsulfonyl, heteroaralkylsulfonyl, arylsulfonyl, heteroarylsulfonyl, nitro, cyano, N-morpholinoalkyl, N-morpholinoalkoxy, N-morpholinoaralkyl, N-morpholinoaralkoxy, N-morpholinoheteroaralkyl, N-morpholinoheteroaralkoxy, N-mono and N,N-dialkylaminoalkyl and N-mono- and N,N-dialkylaminoethoxy, quinuclidinylamino, quinuclidinyloxy, quinuclidinocarbonyl or ureido.

A preferred embodiment of this invention is where R₅ and R₆ are aryl.

Heteroaryl is defined as an unsubstituted or an optionally substituted mono- or bicyclic ring system of about 5 to about 12 carbon atoms and where each monocyclic ring may possess from 0 to about 4 heteroatoms, and each bicyclic ring may possess about 0 to about 5 heteroatoms selected from N, O, and S provided said heteroatoms are not vicinal oxygen and/or sulfur atoms and where the substituents, numbering from 0 to about 5 may be located at any appropriate position of the ring system and are described by R₁₁.

Examples of such mono- and bicyclic ring systems which are by no means meant to limit the scope of this invention, include benzofuran, benzothiophene, indole, benzopyrazole, coumarin, isoquinoline, pyrrole, thiophene, furan, thiazole, imidazole, pyrazole, triazole, quinoline, pyrollidenone, pyrimidine, pyridine, pyridone, pyrazine, pyridazine, isothiazole, isoxazole and tetrazole.

The pharmaceutically acceptable salts include both acid and base addition salts.

The term acid addition salts refers to those salts which retain the biological effectiveness and properties of the free bases and which are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like.

The term base addition salts include those derived from inorganic bases such as sodium, potassium, lithium, ammonium, calcium, magnesium, iron, zinc, copper, manganese, aluminum salts and the like. Particularly preferred are the ammonium, potassium, sodium, calcium and magnesium salts derived from pharmaceutically acceptable organic non-toxic bases which include salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, trimethamine, dicyclohexylamine, lysine, arginine, histidine, caffeine, procaines, hydrabamine, choline, betaine, ethylenediamine, glucosamine, methylglucamine, theobromine, purines, piperazine, piperidine, N-ethylpiperidine, polyamine resins and the like. Particularly preferred organic non-toxic bases are isopropylamine, diethylamine, ethanolamine, trimethamine, dicyclohexylamine, choline and caffeine.

As employed above and throughout the disclosure, the following terms, unless otherwise indicated, shall be understood to have the following meanings:

Alkyl is defined as a saturated aliphatic hydrocarbon which may be either straight- or branched-chain or cyclic. Preferred groups have no more than about 12 carbon atoms and may be methyl, ethyl and structural isomers of propyl, butyl, pentyl, hexyl, heptyl, octyl, nonyl, decyl, undecyl, dodecyl, cyclopropyl, cyclobutyl, cyclopentyl, cyclohexyl, cycloheptyl and cyclooctyl.

Aryl is defined as a phenyl or naphthyl or a substituted phenyl and a substituted naphthyl ring wherein one or more of the hydrogens has been replaced by the same or different substituents as selected from R₁₁.

Alkoxy refers to an alkyl-O-group, e.g. methoxy or ethoxy.

Aryloxy refers to an aryl-O-group, e.g. phenoxy.

Heteroxy refers to a hetero-O-group, e.g. 4-pyridyloxy.

Aralkyl refers to an alkyl group substituted by an aryl radical, e.g. benzyl.

Heteroaralkyl refers to an alkyl group substituted by a heteroaryl radical, e.g. (4-pyridyl)methyl.

Alkenyl is defined as an unsaturated aliphatic hydrocarbon which may be either straight- or branched-chain or cyclic. Preferred groups have no more than about 12 carbon atoms and no fewer than 2 carbon atoms and contain from one to up to about 6 double bonds. Examples of alkenyl groups include ethenyl, propenyl, 1-hexenyl, 1,3-hexdienyl, 2-methyl-2-butenyl, 2-methyl-3-pentenyl, cyclopentenyl, cyclohexenyl and cyclobutenyl.

Alkylacyl refers to an alkyl-C(O)-group, e.g. acetyl or propionyl.

Alkylacyloxy refers to an alkyl-C(O)O-group, e.g. an acetoxy group.

Alkylacylamino means alkyl-C(O)-NR₇ where R₇ has been defined previously.

Alkylacylguanidino means alkyl-C(O)NR₆C(NR₇)NH- where R₆ and R₇ have been defined previously.

Ureido refers to an R₆R₇N-C(O)-N-R₆-group where R₆ and R₇ are described previously.

Haloalkyl is defined as a saturated aliphatic hydrocarbon of 1-12 carbon atoms which may be either straight- or branched-chain or cyclic and where one or more of the hydrogen atoms is replaced with halogen. Preferred haloalkyl groups include trifluoromethyl and pentafluoroethyl.

Halo means bromo, chloro and fluoro.

The present invention also concerns a method of inhibiting interleukin-1β protease activity in a mammal in need of such treatment comprising administering to said mammal an effective inhibitory amount of a compound of formula (I) or a pharmaceutical composition containing a compound of the formula (I) in a pharmaceutically acceptable carrier. The method of inhibition is directed for the treatment of IL-1β mediated disease states or disorders which include: infectious diseases, such as meningitis and salpingitis; septic shock, respiratory diseases; inflammatory conditions, such as arthritis, cholangitis, colitis, encephalitis, endocerolitis, hepatitis, pancreatitis and reperfusion injury, immune-based diseases, such as hypersensitivity; auto-immune diseases, such as multiple sclerosis; bone diseases; and certain tumors.

The pharmaceutical composition of the present invention comprises an active ingredient of the compound of formula (I) in admixture with a pharmaceutically acceptable, non-toxic carrier. Such compositions may be prepared for use by any of a variety of routes depending upon the specific end use, including parenterally (including subcutaneous, intra-articular, intramuscular and intravenous administration), particularly in the form of liquid solutions or suspensions; for oral or buccal (including sublingual) administration, particularly in the form of tablets or capsules; intranasally, particularly in the form of powders, nasal drops or aerosols or transdermally. The most suitable route in any given case will depend upon the use, the particular active ingredient, and the subject involved. The compound or composition may also be administered by means of controlled-release, depot implant or injectable formulations as described more fully herein.

When administered orally (or rectally) the ccmpounds will usually be formulated into a unit dosage form such as a tablet, capsule, suppository or cachet. Such formulations typically include a solid, semisolid or liquid carrier or diluent. Exemplary diluents and vehicles are lactose, dextrose, sucrose, sorbitol, mannitol, starches, gum acacia, calcium phosphate, mineral oil, cocoa butter, oil of theobroma, alginates, tragacanth, gelatin, syrup, methylcellulose, polyoxyethylene sorbitan monolaurate, methyl hydroxybenzoate, propyl hydroxybenzoate, talc, and magnesium stearate.

The compositions may be prepared by any of the methods well-known in the pharmaceutical art, for example as described in Remington's Pharmaceutical Sciences, 17th edition, Mack Publishing Company, Easton, PA, 1985. Formulations for parenteral administration may contain as common excipients sterile water or saline, alkylene glycols such as propylene glycol, polyalkylene glycols such as polyethylene glycol, oils of vegetable origin, hydrogenated naphthalenes and the like. Examples of vehicles for parenteral administration include water, aqueous vehicles such as saline, Ringer's solution, dextrose solution, and Hank's solution and nonaqueous vehicles such as fixed oils (such as corn, cottonseed, peanut, and sesame), ethyl oleate, and isopropyl myristate. Sterile saline is a preferred vehicle and the compounds are sufficiently water soluble to be made up as a solution for all foreseeable needs. The vehicle may contain minor amounts of additives such as substances that enhance solubility, isotonicity, and chemical stability, for example, antioxidants, buffers, and preservatives. For oral administration, the formula can be enhanced by the addition of bile salts and also by the addition of acylcarnitines (Am. J. Physiol. 251:332 (1986)). Formulations for nasal administration may be solid and contain as excipients, for example, lactose or dextran, or may be aqueous or oily solutions for administration in the form of nasal drops or metered spray. For buccal administration typical excipients include sugars, calcium stearate, magnesium stearate, pregelatinated starch, and the like.

When formulated for nasal administration the absorption across the nasal mucous membrane is enhanced by surfactant acids, such as for example, glycocholic acid, cholic acid, taurocholic acid, ethocholic acid, desoxycholic acid, chenodesoxycholic acid, dehydrocholic acid, glycodeoxycholic acid, and the like (See, B.H. Vickery, "LHRH and its Analogs-Contraception and Therapeutic Applications", Pt. 2. B.H. Vickery and J.S. Nester, Eds., MTP Press, Lancaster, UK, 1987).

In general, for the uses as described in the present invention, it is expedient to administer the active ingredient in amounts between about 0.1 and 100 mg/kg body weight, most preferably from about 0.1 to 30 mg/kg body weight for human therapy, the active ingredient being administered preferably in the range of from about 0.1 to about 20-50 mg/kg/day. This administration may be accomplished by a single administration, by distribution over several applications or by slow release in order to achieve the most effective results. When administered as a single dose, administration will most preferably be in the range of from about 0.1 mg/kg to about 10 mg/kg.

The exact dose and regimen for administration of these compounds and compositions will necessarily be dependent upon the needs of the individual subject being treated, the type of treatment, and the degree of affliction or need. In general, parenteral administration requires lower dosage than other methods of administration which are more dependent upon absorption.

The compounds of this invention are prepared by one of two related general synthetic methods as described in Schemes 1 and 2. Referring to Scheme 1, the first step of the method involves the synthesis of Z-protected amino acid bromomethyl ketones (formula 2), where the "Z-group" refers to the "benzyloxycarbonyl group". Methods for the preparation of various Z-protected aspartic acids and aspartic acid-containing peptides (formula 1) which are used as the starting materials for the synthesis of the bromomethyl ketones (formula 2), are well established in the art. ("The Peptides" E. Gross and J. Meienhofer, Eds. Academic Press, Orlando, FL.; 1979; Vol. 1 - 3.) The Z-protected amino acids, dipeptides, and polypeptides (formula 1), which in some cases are commercially available, are then converted to the aspartic acid-containing bromomethylketones (formula 2), by way of hydrobromination of a diazomethyl ketone intermediate. This is accomplished by methods described in Shaw, E. and Ruscica, J., Biol. Chem., 1968, 243, 6312 and Green, E.D.J. and Shaw, E., J. Biol. Chem., 1981, 256, 1923.

The t-butyl ester bromoketone (formula 2) is reacted with a variety of phosphinic acids. This is conducted by exposing the bromomethyl ketone to an excess of the phosphinic acids in a DMF containing sodium or potassium hydride or potassium fluoride. The reaction can be conveniently monitored by thin layer chromatography (TLC) and once the TLC indicates that the displacement of the bromide with the phosphinic acids is completed, the product is isolated using standard procedures. The desired aspartic acid-based mono-t-butyl ester phosphinyloxymethyl ketones (formula 3) may be purified by conventional methods including recrystallization and silica gel column chromatography. 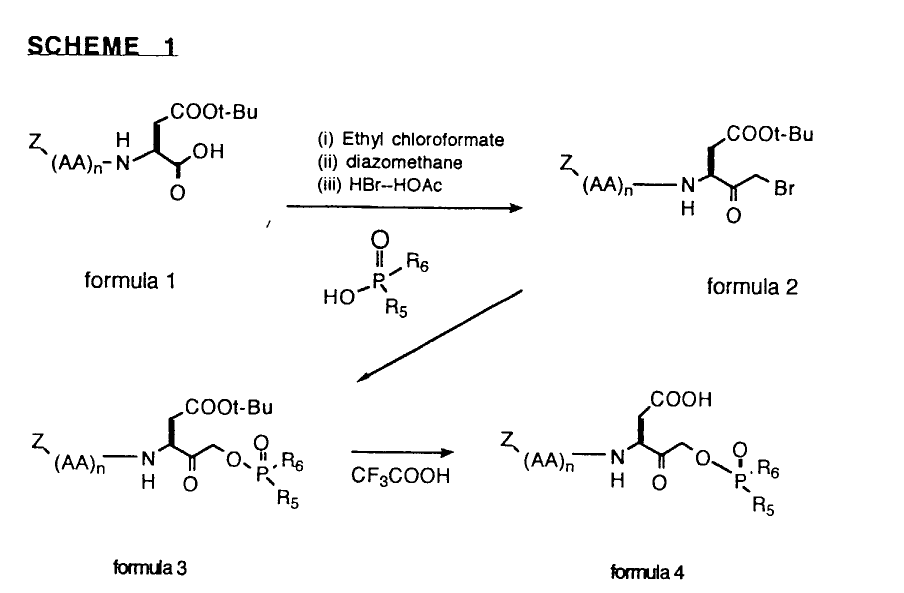 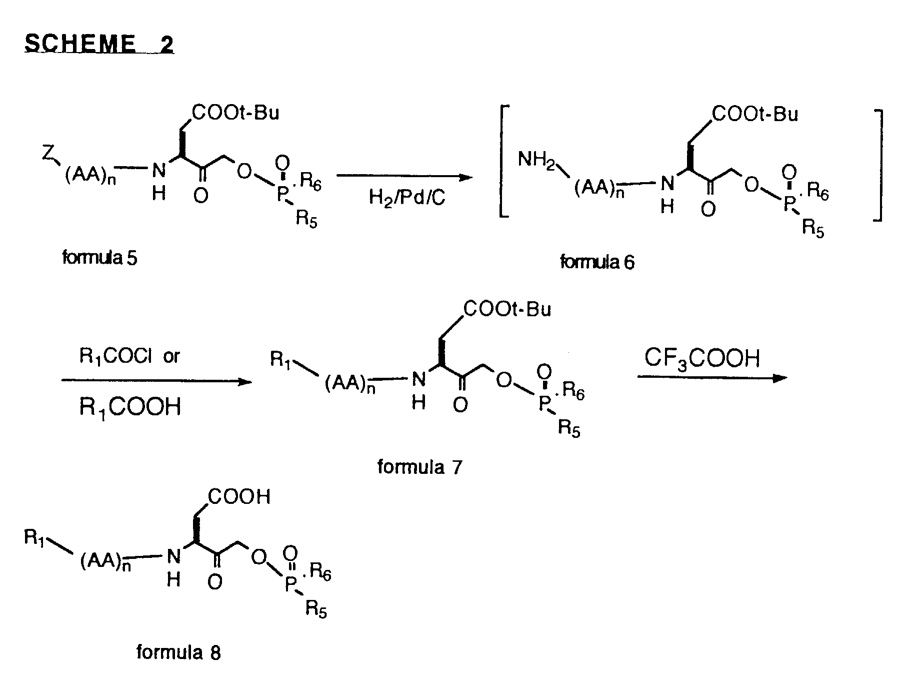 wherein
AA, R₁, R₅, and R₆ are as defined in formula (I) and Z is defined as the benzyloxycarbonyl group.

The remaining synthetic transformation to generate the ICE inhibitors is hydrolysis of the t-butyl ester function. This is conducted by exposing the ester to a 25% solution of trifluoroacetic acid at 25°C The de-esterification is usually complete within 3 h and the removal of the volatile TFA and solvent affords the aspartic acid derivative in formula 4. The yield of the reaction is quantitative in most instances, providing the t-butyl ester starting material of high purity. Purification, if required, can be performed by recrystallization or chromatographic techniques which are well known to those skilled in the art. A solution of 3M anhydrous HCl in ethyl acetate may be used in place of TFA-methylene chloride solution with equal efficiency.

In Scheme 2, the synthesis of phosphinyloxymethyl ketones which possess an N-terminal group (other than the Z group) are described. The aspartic acid derivatives of formula 5 are the starting material for the synthesis of these compounds. The Z group is removed to generate an N-terminal amine (formula 6) under hydrogenolytic conditions. The reagents and conditions used to carry out the hydrogeneration reaction are hydrogen gas, ambient temperature and pressure, 5%-Pd/C as the catalyst in an alcoholic solvent (ethanol), optionally containing 2 equivalents of hydrochloric acid.

The N-terminal amine is then condensed with a carboxylic acid chloride or an active carboxylic acid ("The Practice of Peptide Synthesis", M Bodanszky, Springer-Verlag, NY,1984) to yield an amide (formula 7). Lastly, the t-butyl ester is removed with trifluoroacetic acid to afford the aspartic acid derivative (formula 8).

The phosphinic acids used in the reaction with the bromomethyl ketones can be either purchased from commercial sources or synthesized by adopting known procedures. Their synthesis is readily deduced by those skilled in the art of organic synthesis.

The invention will now be illustrated with reference to the following examples but is in no way to be construed as limited thereto.

### Example 1

### N-[4-(N,N-Dimethylaminomethy)]benzoyl-L-valyl-L-aspartic acid diphenylphosphinyloxymethyl ketone

Part A: N-Benzyloxycarbonyl-L-valine-L-aspartic acid bromomethyl ketone β-tert butyl ester (1.16 mmol; Formula 2) was dissolved in 2 ml DMF containing diphenylphosphinic acid (1.4 mmol) and powdered anhydrous KF (1.6 mmol). The reaction mixture was stirred under N₂ for 16 hrs. The mixture was diluted with water (30 ml), extracted with ether (3x20 ml), and the organic layer was washed with 0.1 N NaOH (3x10 ml) followed by brine. The ether solution was dried over magnesium sulfate and concentrated in vacuo to afford (80%) of the β-tert-butyl ester (formula 3) as a tan solid.
Part B: N-Benzyloxycarbonyl-L-valine-L-aspartic acid diphosphinyloxymethyl ketone β-tert-butyl ester (2 mmol; Part A above) was dissolved in absolute ethanol (100 ml) containing 2 equiv. of 6 N aqueous HCI (4 mmol) and a catalytic amount of 10% palladium on carbon. The reaction mixture was stirred under an ambient atmosphere of H₂ gas for about 1hr. The solution was filtered and the solvent was removed in vacuo to give the corresponding HCl-salt (formula 6) which was used immediately in the subsequent reaction.
Part C: The HCl-salt obtained in Part B above was dissolved in CH₂Cl₂ (10 ml), cooled to -20°C and N-[4-(N,N-dimethylaminomethyl)]benzoyl chloride (4 mmol) was added followed by the addition of 10 mg of dimethylamino pyridine (DMAP) and N-methylmorpholine (5 mmol). The reaction mixture was stirred for 2 hrs at 25°C. The solvent was removed in vacuo and the residue was dissolved in EtOAc (10 ml) which was then washed with water, 0.01 N aqueous HCI, saturated NaHCO₃, brine and dried over MgSO₄. The EtOAc was removed in vacuo and the residue was purified by silica gel chromatography (CH₂Cl₂-MeOH) to obtain N-[4-(N,N-dimethylaminomethy)]benzoyl-L-valine-L-aspartic acid diphenylphosphinyloxymethyl ketone β-tert-butyl ester (formula 7) in 50% yield.
Part D: The β-tert-butyl ester obtained in Part C above (1 mmol) was dissolved in trifluoroacetic acid - CH₂Cl₂ (1:4) and the solution was stirred for 2 hrs at 25°C. The solvent was removed in vacuo and the residue was triturated with ether. The white solid was collected and dried to give the title compound in 90% yield. Mass spectrum m/z=608 [M + H].

The 4-(N,N-dimethylaminomethyl) benzoyl chloride was prepared by reacting the acid with excess oxalyl chloride for 1 hr at 25°C. The 4-(N,N-dimethylaminomethyl) benzoic acid was in turn prepared from methyl 4-aminomethylbenzoate via reductive alkylation (CH₂O, Na(OAc)₃BH as in J. Org. Chem.,1972, 37, 1673) followed by hydrolysis using 10% aqueous NaOH.

Following the procedure described in Schemes 1 and 2 and by analogy to Example 1, the following compounds were prepared.

### Example 2

### N-Benzyloxycarbonyl-L-valyl-L-aspartic acid diphenylphosphinyloxymethyl ketone

Mass spectrum: m/z = 581 [M + H]

### Example 3

### N-Benzyloxycarbonyl-L-aspartic acid diphenylphosphinyloxymethyl ketone.

Mass spectrum: m/z = 596 [M+H]

### Example 4

### N-Benzyloxycarbonyl-L-aspartic acid (p-chlorophenyl)phenylphosphinyloxymethyl ketone.

Mass spectrum: m/z = 516 [M+H]

### Example 5

### N-Benzyloxycarbonyl-L-aspartic acid (p-methoxyphenyl)phenylphosphinyloxymethyl ketone.

| Anal. for C₃₁H₃₅N₂O₉P ^{.} 0.5 CF₃CO₂H ^{.} 0.5H₂O: | | | | | | |
|---|---|---|---|---|---|---|
| Calcd. | C, | 56.80; | H, | 5.44; | N, | 4.14. |
| Found: | C, | 56.85; | H, | 5.33, | N, | 4.15. |

### Example 6

### N-Benzyloxycarbonyl-L-valyl-L-alanyl-L-aspartic acid diphenylphosphinyloxymethylketone.

Mass spectrum: m/z = 652 [M+H]

### Example 7

### N-[4-(N,N-Dimethylaminomethyl)]benzoyl-L-valyl-L-alanyl-L-aspartic acid diphenylphosphinyloxymethylketone.

Mass spectrum: m/z = 679 [M+H]

### Example 8

### N-Benzyloxycarbonyl-L-valyl-L-aspartic acid (p-methoxyphenyl)phenylphosphinyloxymethyl ketone.

Mass spectrum: m/z = 611 [M+H]

### Example 9

### N-Benzyloxycarbonyl-L-valyl-L-aspartic acid (p-chlorophenyl)phenylphosphinyloxymethyl ketone.

Mass spectrum: m/z = 616 [M+H]

### Example 10

### N-Benzyloxycarbonyl-L-aspartic acid di-(p-methoxyphenyl)phosphinyloxymethyl ketone.

| Anal. . for C₂₇H₂₈NO₉P · 0.5 CF₃CO₂H: | | | | | | |
|---|---|---|---|---|---|---|
| Calcd | C, | 56.19; | H, | 4.80; | N, | 2.34. |
| Found: | C, | 55.98, | H, | 4.77; | N, | 2.38. |

### Example 11

### N-Benzyloxycarbonyl-L-aspartic acid (m-methoxyphenyl)phenylphosphinyloxymethyl ketone.

| Anal. for C₂₆H₂₆NO₈P · 0.5 CF₃CO₂H: | | | | | | |
|---|---|---|---|---|---|---|
| Calcd. | C, | 57.05; | H, | 4.70; | N, | 2.46. |
| Found: | C, | 57.29; | H, | 4.78; | N, | 2.50. |

### Example 12

### N-4-(Pyridyl)carbomethoxy-L-valyl-L-alanyl-L-aspartic acid diphenylphosphinyloxymethyl ketone.

Mass spectrum: m/z = 767 [M+H]

### Example 13

### N-Benzyloxycarbonyl-L-valyl-D-aspartic acid diphenylphosphinyloxymethyl ketone

Mass spectrum: m/z = 581 [M+H]

### Example 14

### N-3-(Quinuclidinyl)carbonyl-L-valyl-L-alanyl-L-aspartic acid diphenylphosphinyloxymethyl ketone.

Mass spectrum: m/z = 769 [M+H]

### Example 15

### N-Benzyloxycarbonyl-L-valyl-L-aspartic acid dimethylphosphinyloxymethyl ketone

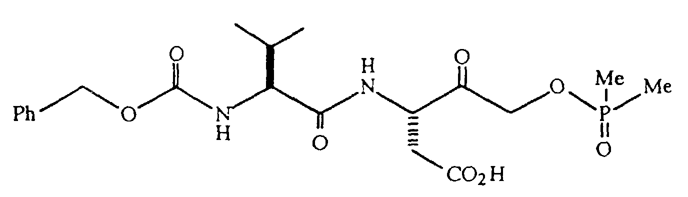

Mass Spectrum m/z = 457 [M+H]

### Example 16

### N-Benzyloxycarbonyl-L-valyl-L-aspartic acid (methyl)(4-(2-methylpropyl)phenyl)phosphinyloxymethyl ketone

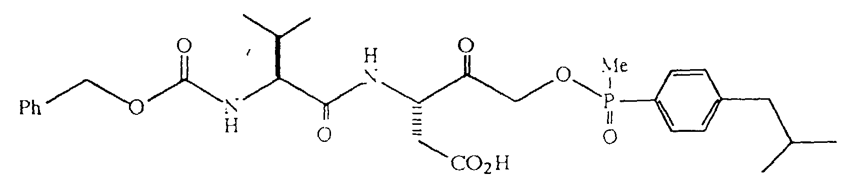

Mass Spectrum m/z = 589 [M+H]

### Example 17

### N-Benzyloxycarbonyl-L-valyl-L-aspartic acid (phenyl)((4-phenyl)phenyl)phosphinyloxymethyl ketone

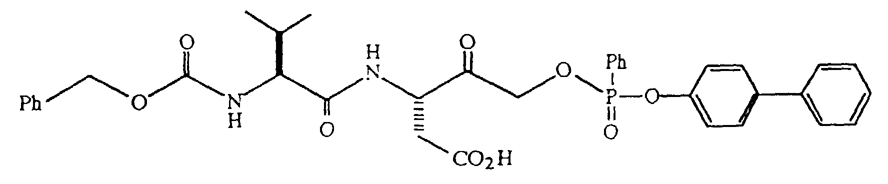

Mass Spectrum m/z = 678 [M+H]

### Example 18

### N-Benzyloxycarbonyl-L-valyl-L-aspartic acid phenylphosphinyloxymethyl ketone

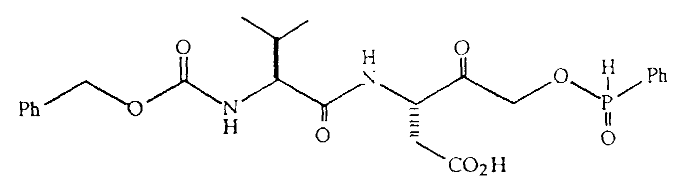

Mass Spectrum m/z = 589 [M+H]

### Example 19

### N-Benzyloxycarbonyl-L-valyl-L-aspartic acid (methyl)((4-phenyl)phenyl)phosphinyloxymethyl ketone

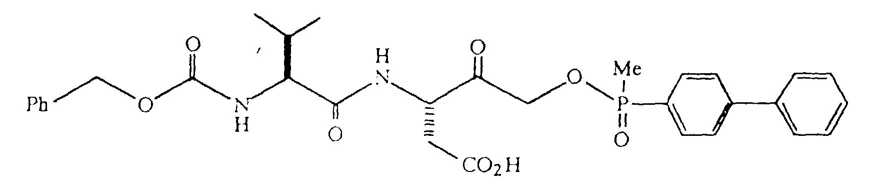

Mass Spectrum m/z = 609 [M+H]

Compounds of the present invention were tested for IL-1β protease inhibition activity according to the following protocol:

Partially purified IL-1β protease is stored at -80°C, thawed on ice, and preincubated for 10 minutes at 37°C with 2.5 mM dithiothreitol in a buffer solution containing 10 mM Tris-HCI (pH 8.0) and 25% (v/w) glycerol. Inhibitors are prepared as stock solutions in dimethyl sulfoxide (DMSO). The protease is preincubated with inhibitor in a volume of 20 µL in a 1.5 ml polypropylene microcentrifuge tube for 15 minutes at 37°C. The volume of compound added to the assay is adjusted to yield a DMSO concentration in the preincubation of <15% (v/v). The enzyme assay is then initiated by the addition of substrate (TRITC-AYVHDAPVRS-NH₂) to yield a final concentration of 67 µM in a final volume of 30 µl. The reactions are carried out for 60 minutes at 37°C in the dark and are terminated by the addition of 10 µl 10% trifluoroacetic acid (TFA). Following the addition of 115 µl 0.1% TFA, the samples are analyzed by high pressure liquid chromatography using a reverse phase (C18) column and elution with an acetonitrile/water/TFA gradient. Substrate and product are monitored by their absorbance at 550 nm and elute at 4.2 and 5.2 minutes, respectively.

The IC50 values recorded for inhibition against the enzyme were <10 µM.

## Claims

1. A compound of the formula (I) or a pharmaceutically acceptable salt thereof: 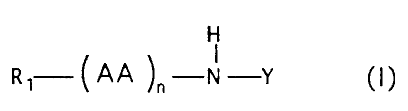 wherein:
n is 0-4;
Y is 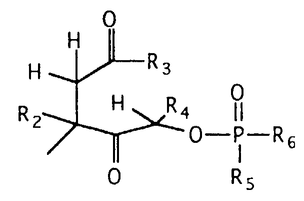
and when R₃ is OH, then Y can also be 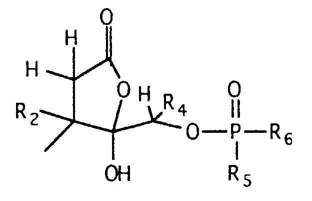
R₂ is H or deuterium;
R₃ is OH, OR₇, NR₇OR₈ or NR₇R₈;
where R₇ and R₈ are independently H, alkyl, cycloalkyl, aralkyl, heteroaralkyl, aryl or heteroaryl;
R₄ is H or lower alkyl;
R₅ and R₆ are optionally and independently selected from H, OH, alkyl, aryl, aralkyl, heteroaryl, heteroaralkyl, alkoxy, aroxy, heteroaroxy, aralkoxy, heteroaralkoxy, alkenyl, aralkenyl or heteroaralkenyl;
AA is independently selected from the group consisting of (a) and (b) where (a) is defined as an amino acid of formula II 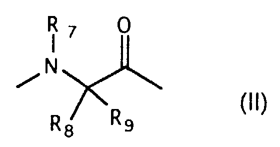
wherein R₇ and R₈ are defined as above and R₉ is (CR₆R₇)₀₋₆-R₁₀;
where R₁₀ is a radical optionally selected from R₁₁,
where R₁₁ is described below; and
where group (b) is selected from the group consisting of: 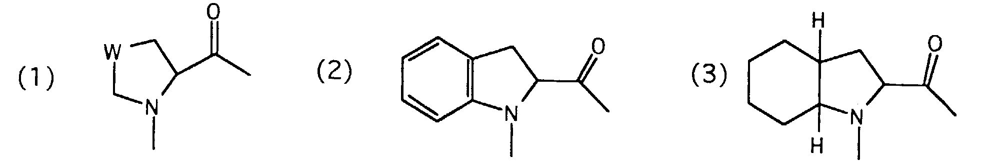 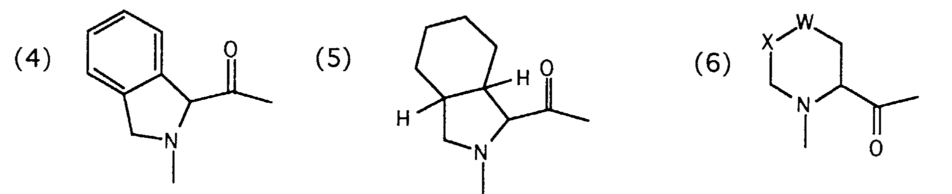 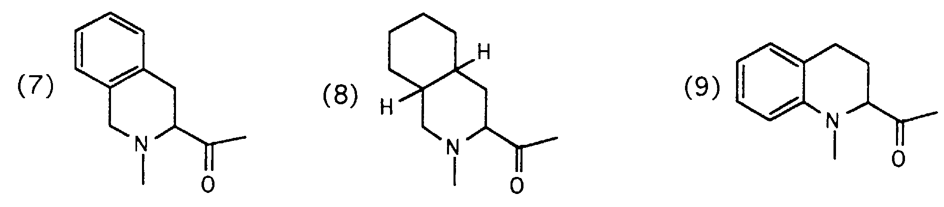 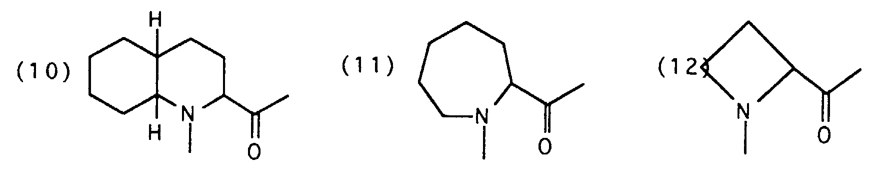 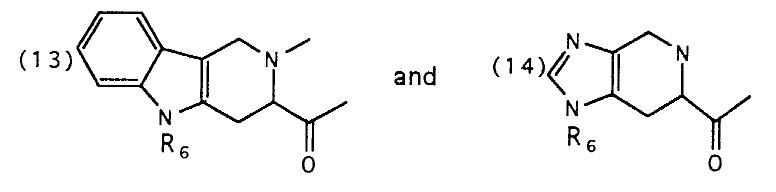 where W and X are optionally CH₂, O, S or NR₇;
R₁ is R₁₀-CO- or R₁₀SO₂-, where R₁₀ is defined previously;
R₁₁ is H, alkyl, alkenyl, aryl, heteroaryl, aralkyl, heteroaralkyl, aralkenyl, heteroaralkenyl, hydroxy, alkoxy, 2-(alkoxy)ethoxy, 2-(alkoxy)aminoethyl, 2-(alkoxy)-N-alkylaminoethyl, aralkoxy, heteroaralkoxy, alkylacyloxy, aralkylacyloxy, heteroaralkylacyloxy, aracyloxy, heteroaracyloxy, aryloxyalkylacyloxy, heteroaryloxyalkylacyloxy, alkylacyl, aralkylacyl, heteroaralkylacyl, alkylacylamino, aralkylacylamino, heteroaralkylacylamino, aracylamino, heteroaracylamino, aryloxyalkylacylamino, heteroaryloxyalkylacylamino, alkyloxyalkylacylamino, alkoxyacylamino, aralkoxyacylamino, heteroaralkoxyacylamino, aracyl, heteroaracyl, aryloxyalkylacyl, heteroaryloxyalkylacyl, halo, haloalkyl, guanidino, -mono- and di-alkylguanidino, mono- and di-aralkyl-guanidino, mono- and di-heteroaralkylguanidino, alkylacylguanidino, aralkylacylguanidino, heteroaralkylguanidino, aracylguanidino, heteroarylguanidino, amidino, mono- and di-alkylamidino, mono- and diaralkylamidino, mono- and di-heteroaralkylamidino, amino, mono- and dialkylamino, mono- and diaralkylamino, mono- and di-heteroaralkylamino, carboxy, alkylcarboxy, carbalkoxy, carbaralkoxy, carbheteroaralkoxy, carbalkoxyalkenyl, carboxamido, mono- and dialkylcarboxamido, mono- and diarcarboxamido, mono and di-heteroarcarboxamido, mono- and di-aralkylcarboxamido, mono- and di-heteroaralkylcarboxamido, thio, alkylthio, arylthio, heteroarylthio, aralkylthio, heteroaralkylthio, sulfonamido, mono- and di-alkylsulfonamido, mono- and di-aralkylsulfonamido, mono- and di-heteroaralkylsulfonamido, morpholinosulfonamido, alkylsulfonyl, aralkylsulfonyl, heteroaralkylsulfonyl, arylsulfonyl, heteroarylsulfonyl, nitro, cyano, N-morpholinoalkyl, N-morpholinoalkoxy, N-morpholinoaralkyl, N-morpholinoaralkoxy, N-morpholinoheteroaralkyl, N-morpholinoheteroaralkoxy, N-mono and N,N-dialkylaminoalkyl and N-mono- and N,N-dialkylaminoethoxy, quinuclidinylamino, quinuclidinyloxy, quinuclidinocarbonyl or ureido;
It being understood that:
alkyl designates a saturated aliphatic hydrocarbon which may be either straight- or branched-chain or cyclic and contain no more than 12 carbon atoms;
aryl designates phenyl or naphthyl optionally substituted by one or more substituents which are independently selected from the meanings of R₁₁;
heteroaryl designates an optionally substituted mono- or bicyclic ring system of from 5 to 12 carbon atoms, in which each monocyclic ring possesses from 0 to 4 heteroatoms and each bicyclic ring possesses from 0 to 5 heteroatoms selected from N, O and S provided that said heteroatoms are not vicinal oxygen and/or sulfur atoms, the substituents which are described by R₁₁ being located at any position of the ring system;
alkenyl designates an unsaturated aliphatic hydrocarbon which may be either straight- or branched chain or cyclic and contain from 2 to 12 carbon atoms and from one to 6 double bonds;
alkylacyl designates an alkyl-C(O)- group; and
ureido designates an R₆R₇N-C(O)-NR₆ group in which R₆ and R₇ are as defined above.

2. A compound according to claim 1 wherein R₅ and R₆ are aryl in which aryl is as defined in claim 1.

3. A compound according to claim 1 selected from the group consisting of: N-[4-(N,N-Dimethylaminomethyl)]benzoyl-L-valyl-L-aspartic add diphenylphosphinyloxymethyl ketone,
N-Benzyloxycarbonyl-L-valyl-L-aspartic add diphenylphosphinyloxymethyl ketone,
N-Benzyloxycarbonyl-L-aspartic acid diphenylphosphinyloxymethyl ketone,
N-Benzyloxycarbonyl-L-aspartic acid (p-chlorophenyl)phenylphosphinyloxymethyl ketone and
N-Benzyloxycarbonyl-L-aspartic acid (p-methoxyphenyl)phenylphosphinyloxymethyl ketone.

4. A compound according to claim 1 selected from the group consisting of: N-Benzyloxycarbonyl-L-valyl-L-alanyl-L-aspartic acid diphenylphosphinyloxymethylketone,
N-[4-(N,N-Dimethylaminomethyl)]benzoyl-L-valyl-L-alanyl-L-aspartic acid diphenylphosphinyloxymethylketone,
N-Benzyloxycarbonyl-L-valyl-L-aspartic acid (p-methoxyphenyl)phenylphosphinyloxymethyl ketone,
N-Benzyloxycarbonyl-L-valyl-L-aspartic acid (p-chlorophenyl)phenylphosphinyloxymethyl ketone and
N-Benzyloxycarbonyl-L-aspartic acid di-(p-methoxyphenyl)phosphinyloxymethyl ketone.

5. A compound according to daim 1 selected from the group consisting of: N-Benzyloxycarbonyl-L-aspartic acid (m-methoxyphenyl)-phenylphosphinyloxymethyl ketone,
N-4-(Pyridyl)carbomethoxy-L-valyl-L-alanyl-L-aspartic acid diphenylphosphinyloxymethyl ketone,
N-Benzyloxycarbonyl-L-valyl-D-aspartic acid diphenylphosphinyloxymethyl ketone and
N-3-(Quinuclidinyl)carbonyl-L-valyl-L-alanyl-L-aspartic acid diphenylphosphinyloxymethyl ketone.

6. A compound according to claim 1 selected from the group consisting of: N-Benzyloxycarbonyl-L-valyl-L-aspartic acid dimethylphosphinyloxymethyl ketone,
N-Benzyloxycarbonyl-L-valyl-L-aspartic acid (methyl)(4-(2-methylpropyl)phenyl)phosphinyloxymethyl ketone,
N-Benzyloxycarbonyl-L-valyl-L-aspartic acid (phenyl)((4-phenyl)-phenyl)phosphinyloxymethyl ketone,
N-Benzyloxycarbonyl-L-valyl-L-aspartic acid phenylphosphinyloxymethyl ketone and
N-Benzyloxycarbonyl-L-valyl-L-aspartic acid (methyl)((4-phenyl)-phenyl)phosphinyloxymethyl ketone

7. A pharmaceutical composition for inhibiting interleukin-1β protease comprising a compound of the formula (I) or a pharmaceutically acceptable salt thereof according to any one of the preceding claims in a pharmaceutically acceptable carrier.

8. The use of a compound of formula (I) or a pharmaceutically acceptable salt thereof according to any one of claims 1 to 6 or a pharmaceutical composition according to claim 7 for the preparation of a medicament for inhibiting interleukin-1β protease activity in a mammal in need of such treatment .

## Patentansprüche

1. Verbindung der Formel (I) oder ein pharmazeutisch annehmbares Salz hiervon: 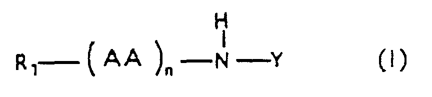 worin bedeuten:
n 0-4;
Y 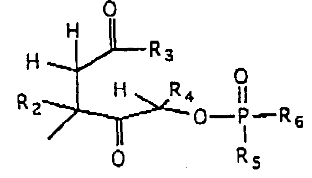
und wenn R₃ OH ist, dann kann Y ebenso 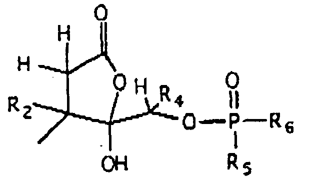 sein:
R₂ H oder Deuterium;
R₃ OH, OR₇, NR₇OR₈ oder Nr₇R₈;
worin R₇ und R₈ unabhängig voneinander H, Alkyl, Cycloalkyl, Aralkyl, Heteroalkyl, Aryl oder Heteroaryl sind;
R₄ H oder Niederalkyl;
R₅ und R₆ wahlweise und unabhängig voneinander gewählt aus H, OH, Alkyl, Aryl, Aralkyl, Heteroaryl, Heteroaralkyl, Alkoxy, Aroxy, Heteroaroxy, Aralkoxy, Heteroaralkoxy, Alkenyl, Aralkenyl oder Heteroaralkenyl;
AA unabhängig gewählt aus der Gruppe, bestehend aus (a) und (b), worin (a) als Aminosäure der Formel II definiert ist; 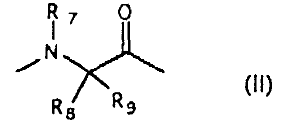
worin R₇ und R₈ wie oben definiert sind und R₉ (CR₆R₇)₀₋₆-R₁₀ ist;
worin R₁₀ ein wahlweise von R₁₁ abgeleiteter Rest ist, worin R₁₁ wie oben beschrieben ist; und
worin die Gruppe (b) aus der Gruppe gewählt ist, bestehend aus: 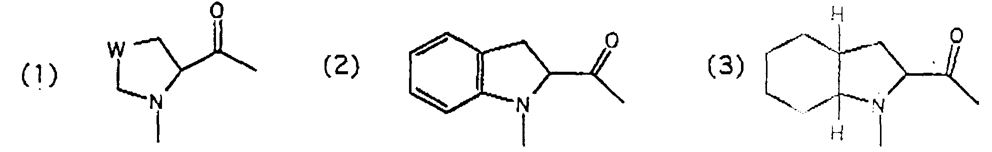 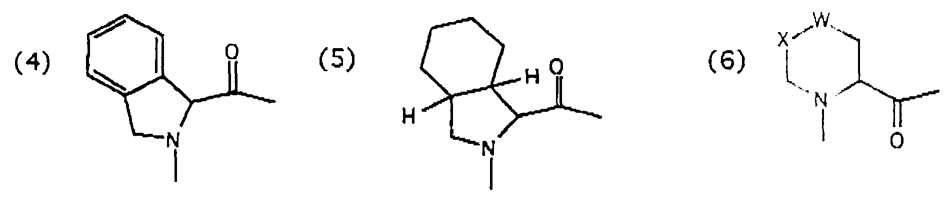 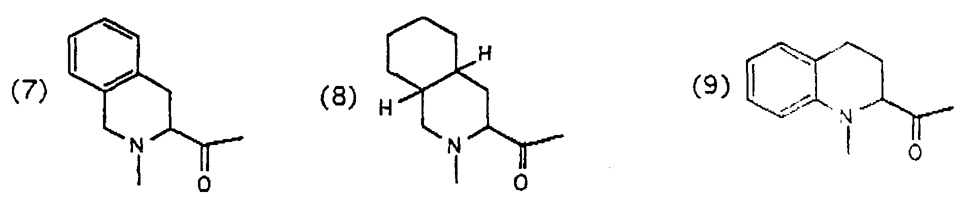 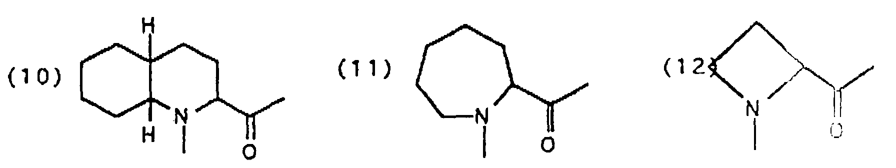 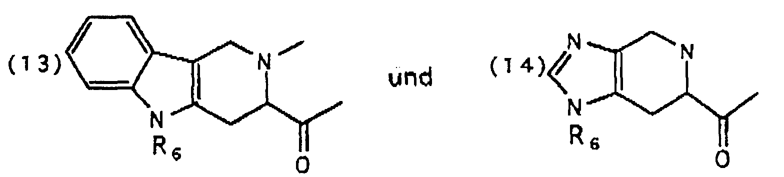 worin W und X wahlweise CH₂, O, S oder NR₇ sind;
R₁ R₁₀-CO- oder R₁₀SO₂-, worin R₁₀ vorangehend definiert ist;
R₁₁ H, Alkyl, Alkenyl, Aryl, Heteroaryl, Aralkyl, Heteroaralkyl, Aralkenyl, Heteroaralkenyl, Hydroxy, Alkoxy, 2-(Alkoxy)ethoxy, 2-(Alkoxy)aminoethyl, 2-(Alkoxy) -N-alkylaminoethyl, Aralkoxy, Heteroaralkoxy, Alkylacyloxy, Aralkylacyloxy, Heteroaralkylacyloxy, Aracyloxy, Heteroaracyloxy, Aryloxyalkylacyloxy, Heteroaryloxyalkylacyloxy, Akylacyl, Aralkylacyl, Heteroaralkylacyl, Akylacylamino, Aralkylacylamino, Heteroaralkylacylamino, Aracylamino, Heteroaracylamino, Aryloxyalkylacylamino, Heteroaryloxyalkylacylamino, Alkoxyalkylacylamino, Alkoxyacylamino, Aralkoxyacylamino, Heteroaralkoxyacylamino, Aracyl, Heteroaracyl, Aryloxyalkylacyl, Heteroaryloxyalkylacyl, Halogen, Halogenalkyl, Guanidino, Mono- und Di-alkylguanidino, Mono- und Di-aralkylguanidino, Mono- und Di-heteroaralkylguanidino, Alkylacylguanidino, Aralkylacylguanidino, Heteroaralkylguanidino, Aracylguanidino, Heteroarylguanidino, Amidino, Mono- und Di-alkylamidino, Mono- und Diaralkylamidino, Mono- und Di-heteroaralkylamidino, Amino, Mono- und Dialkylamino, Mono- und Diaralkylamino, Mono- und Di-heteroaralkylamino, Carboxy, Alkylcarboxy, Carbalkoxy, Carbaralkoxy, Carbheteroaralkoxy, Carbalkoxyalkenyl, Carboximido, Mono- und Dialkylcarboxamido, Mono- und Diarcarboxamido, Mono und Di-heteroarcarboxamido, Mono- und Di-aralkylcarboxamido, Mono- und Di-heteroaralkylcarboxamido, Thio, Alkylthio, Arylthio, Heteroarylthio, Aralkylthio, Heteroaralkylthio, Sulfonamido, Mono- und Di-alkylsulfonamido, Mono- und Di-aralkylsulfonamido, Mono- und Di-heteroaralkylsulfonamido, Morpholinosulfonamido, Alkylsulfonyl, Aralkylsulfonyl, Heteroaralkylsufonyl, Arylsulfonyl, Heteroarylsulfonyl, Nitro, Cyano, N-Morpholinoalkyl, N-Morpholinoalkoxy, N-Morpholinoaralkyl, N-Morpholinoaralkoxy, N-Morpholinoheteroaralkyl, N-Morpholinoheteroaralkoxy, N-Mono- und N,N-Dialkylaminoalkyl und N-Mono- und N,N-Dialkylaminoethoxy, Chinuclidinylamino, Chinuclidinyloxy, Chinuclidinocarbonyl oder Ureido;
wobei von folgendem auszugehen ist:
Alkyl bezeichnet einen gesättigten aliphatischen Kohlenwasserstoff, welcher entweder gerad- oder verzweigtkettig oder cyclisch sein kann und nicht mehr als 12 Kohlenstoffatome enthält;
Aryl bezeichnet Phenyl oder Naphthyl, wahlweise substituiert durch einen oder mehrere Substituenten, welche unabhängig voneinander aus den Bedeutungen für R₁₁ gewählt sind;
Heteroaryl bezeichnet ein wahlweise substituiertes mono- oder bicyclisches Ringsystem mit 5 bis 12 Kohlenstoffatomen, wobei jeder monocyclische Ring 0 bis 4 Heteroatome und jeder bicyclische Ring 0 bis 5 Heteroatome besitzt, gewählt aus N, O und S, mit der Maßgabe, daß die Heteroatome keine vicinalen Sauerstoff- und/oder Schwefelatome sind, wobei die durch R₁₁ beschriebenen Substituenten an irgendeiner Position des Ringsystems angeordnet sind;
Alkenyl bezeichnet einen ungesättigten aliphatischen Kohlenwasserstoff, welcher entweder gerad- oder verzweigtkettig oder cyclisch sein kann und 2 bis 12 Kohlenstoffatome sowie 1 bis 6 Doppelbindungen enthält;
Alkylacyl bezeichnet eine Alkyl-C(O)-Gruppe; und
Ureido bezeichnet eine R₆R₇N-C(O)-NR₆-Gruppe, worin R₆ und R₇ wie oben definiert sind.

2. Verbindung nach Anspruch 1, wobei R₅ und R₆ Aryl sind, worin Aryl wie in Anspruch 1 definiert ist.

3. Verbindung nach Anspruch 1, gewählt aus der Gruppe, bestehend aus: N-[4-(N,N-Dimethylaminomethyl)]benzoyl-L-valyl-L-asparaginsäurediphenylphosphinyloxymethylketon, N-Benzyloxycarbonyl-L-valyl-L-asparaginsäurediphenylphosphinyloxymethylketon, N-Benzyloxycarbonyl-L-asparaginsäurediphenylphosphinyloxymethylketon, N-Benzyloxycarbonyl-L-asparaginsäure(p-chlorophenyl)phenylphosphinyloxymethylketon und N-Benzyloxycarbonyl-L-asparaginsäure(p-methoxyphenyl)phenylphosphinyloxymethylketon.

4. Verbindung nach Anspruch 1, gewählt aus der Gruppe, bestehend aus: N-Benzylcarbonyl-L-valyl-L-alanyl-L-asparaginsäurediphenylphosphinyloxymethylketon, N-[4-(N,N-Dimethylaminomethyl)]bezoyl-L-valyl-L-alanyl-L-asparaginsäurediphenylphosphinyloxymethylketon, N-Benzyloxycarbonyl-L-valyl-L-asparaginsäure(p-methoxyphenyl)phenylphosphinyloxymethylketon, N-Benzyloxycarbonyl-L-valyl-L-asparaginsäure(p-chlorophenyl)phenylphosphinyloxymethylketon und N-Benzyloxycarbonyl-L-asparaginsäuredi-(p-methoxyphenyl)phosphinyloxymethylketon.

5. Verbindung nach Anspruch 1, gewählt aus der Gruppe, bestehend aus: N-Benzyloxycarbonyl-L-asparaginsäure(m-methoxyphenyl)-phenylphosphinyloxymethylketon, N-4-(Pyridyl)carbomethoxy-L-valyl-L-alanyl-L-asparaginsäurediphenylphosphinyloxymethylketon, N-Benzyloxycarbonyl-L-valyl-D-asparaginsäurediphenylphosphinyloxymethylketon und N-3-(Chinuclidinyl)carbonyl-L-valyl-L-alanyl-L-asparaginsäurediphenylphosphinyloxymethylketon.

6. Verbindung nach Anspruch 1, gewählt aus der Gruppe, bestehend aus: N-Benzyloxycarbonyl-L-valyl-L-asparaginsäuredimethylphosphinyloxymethylketon, N-Benzyloxycarbonyl-L-valyl-L-asparaginsäure(methyl)(4-(2-methylpropyl)phenyl)phosphinyloxymethylketon, N-Benzyloxycarbonyl-L-valyl-L-asparaginsäure(phenyl)((4-phenyl)phenyl)phosphinyloxymethylketon, N-Benzyloxycarbonyl-L-valyl-L-asparaginsäurephenylphosphinyloxymethylketon und N-Benzyloxycarbonyl-L-valyl-L-asparaginsäure(methyl)((4-phenyl)phenyl)phosphinyloxymethylketon.

7. Pharmazeutische Zusammensetzung zur Inhibierung von Interleukin-1β-Protease, umfassend eine Verbindung der Formel (I) oder ein pharmazeutisch annehmbares Salz hiervon nach mindestens der vorangehenden Ansprüche in einem pharmazeutisch annehmbaren Träger.

8. Verwendung einer Verbindung der Formel (I) oder eines pharmazeutisch annehmbaren Salzes hiervon nach mindestens einem der Ansprüche 1 bis 6 oder einer pharmazeutischen Zusammensetzung nach Anspruch 7 zur Herstellung eines Arzneimittels zur Inhibierung von Interleukin-1β-Proteaseaktivität in einem Säuger, welcher einer solchen Behandlung bedarf.

## Revendications

1. Composé de formule (I) ou sel pharmaceutiquement acceptable de celui-ci : 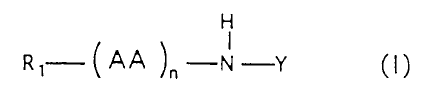 dans laquelle :
n est 0 à 4 ;
Y est 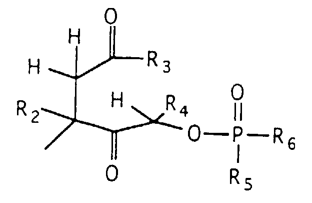
et, si R₃ est OH, alors Y peut aussi être 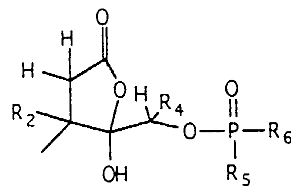
R₂ est H ou le deutérium ;
R₃ est OH, OR₇, NR₇OR₈ ou NR₇R₈, où R₇ et R₈ sont chacun indépendamment H, un groupe alkyle, cycloalkyle, aralkyle, hétéroaralkyle, aryle ou hétéroaryle ;
R₄ est H ou un groupe alkyle inférieur ;
R₅ et R₆ sont facultativement et indépendamment choisis parmi H, OH, un groupe alkyle, aryle, aralkyle, hétéroaryle, hétéroaralkyle, alcoxy, aroxy, hétéroaroxy, aralcoxy, hétéroaralcoxy, alcényle, aralcényle ou hétéroaralcényle ;
AA est choisi indépendamment dans la classe formée par (a) et (b) où (a) est défini comme un acide aminé de formule II 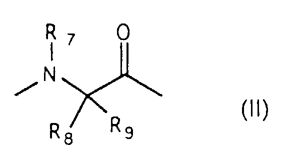
dans laquelle R₇ et R₈ sont tels que définis ci-dessus et R₉ est (CR₆R₇)₀₋₆-R₁₀ où R₁₀ est un radical facultativement choisi parmi R₁₁, R₁₁ étant décrit ci-dessous ; et
le groupe (b) est choisi dans la classe formée par : 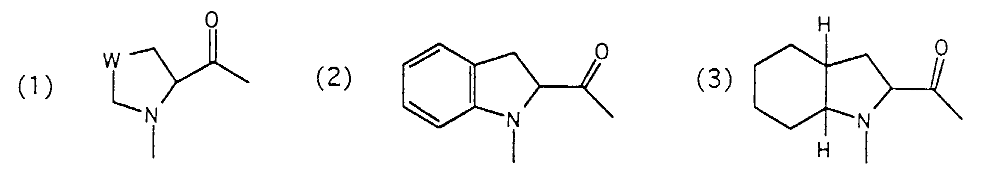 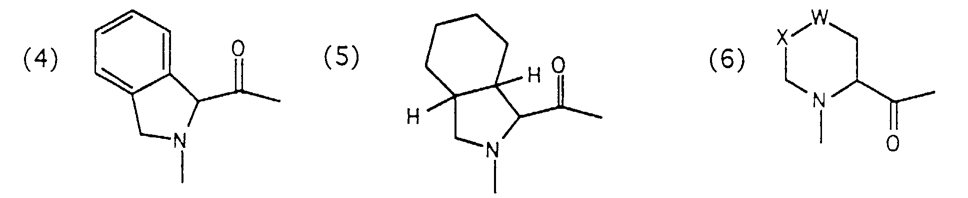 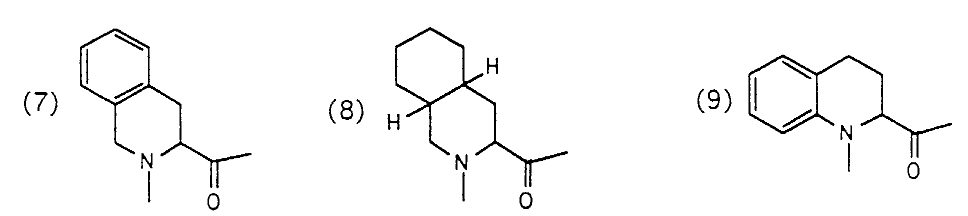 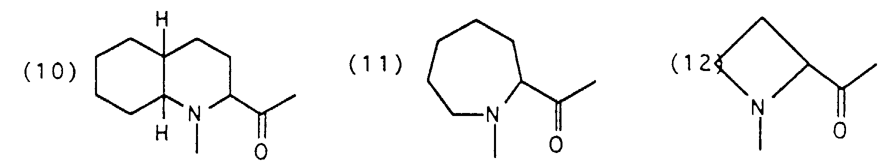 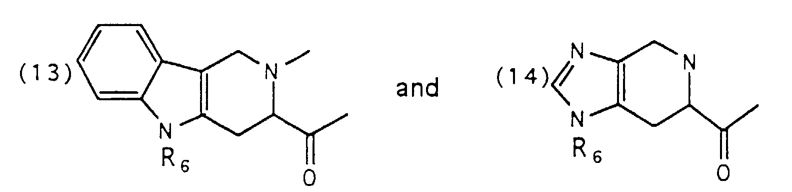
où W et X sont facultativement CH₂, O, S ou NR₇ ;
R₁ est R₁₀-CO- ou R₁₀SO₂-, où R₁₀ est tel que défini précédemment ;
R₁₁ est H, un groupe alkyle, alcényle, aryle, hétéroaryle, aralkyle, hétéroaralkyle, aralcényle, hétéroaralcényle, hydroxyle, alcoxy, 2-(alcoxy)éthoxy, 2-(alcoxy)-aminoéthyle, 2-(alcoxy)-N-alkylaminoéthyle, aralcoxy, hétéroaralcoxy, alkylacyloxy, aralkylacyloxy, hétéroaralkylacyloxy, aracyloxy, hétéroaracyloxy, aryloxyalkylacyloxy, hétéroaryloxyalkylacyloxy, alkylacyle, aralkylacyle, hétéroaralkylacyle, alkylacylamino, aralkylacylamino, hétéroaralkylacylamino, aracylamino, hétéroaracylamino, aryloxyalkylacylamino, hétéroaryloxyalkylacylamino, alcoxyalkylacylamino, alcoxyacylamino, aralcoxyacylamino, hétéroaralcoxyacylamino, aracyle, hétéroaracyle, aryloxyalkylacyle, hétéroaryloxyalkylacyle, halogéno, halogénoalkyle, guanidino, mono- et dialkylguanidino, mono- et diaralkylguanidino, mono- et dihétéroaralkylguanidino, alkylacylguanidino, aralkylacylguanidino, hétéroaralkylguanidino, aracylguanidino, hétéroarylguanidino, amidino, mono- et dialkylamidino, mono- et diaralkylamidino, mono- et dihétéroaralkylamidino, amino, mono- et dialkylamino, mono- et diaralkylamino, mono- et dihétéroaralkylamino, carboxy, alkylcarboxy, carbalcoxy, carbaralcoxy, carbhétéroaralcoxy, carbalcoxyalcényle, carboxamido, mono- et dialkylcarboxamido, mono- et diarcarboxamido, mono- et dihétéroarcarboxamido, mono- et diaralkylcarboxamido, mono- et dihétéroaralkylcarboxamido, thio, alkylthio, arylthio, hétéroarylthio, aralkylthio, hétéroaralkylthio, sulfonamido, mono- et dialkylsulfonamido, mono- et diaralkylsulfonamido, mono- et dihétéroaralkylsulfonamido, morpholinosulfonamido, alkylsulfonyle, aralkylsulfonyle, hétéroaralkylsulfonyle, arylsulfonyle, hétéroarylsulfonyle, nitro, cyano, N-morpholinoalkyle, N-morpholinoalcoxy, N-morpholinoaralkyle, N-morpholinoaralcoxy, N-morpholinohétéroaralkyle, N-morpholinohétéroaralcoxy, N-mono- et N,N-dialkylaminoalkyle et N-mono- et N,N-dialkylaminoéthoxy, quinuclidinylamino, quinuclidinyloxy, quinuclidinocarbonyle ou uréido ; étant entendu que :
le terme "alkyle" désigne un hydrocarbure aliphatique saturé qui peut être à chaîne droite ou ramifiée, ou cyclique, et ne contient pas plus de 12 atomes de carbone ;
le terme "aryle" désigne un groupe phényle ou naphtyle facultativement substitué par un ou plusieurs substituants qui sont choisis indépendamment parmi les valeurs de R₁₁ ;
le terme "hétéroaryle" désigne un système mono- ou bicyclique facultativement substitué de 5 à 12 atomes de carbone, chaque système monocyclique possédant 0 à 4 hétéroatomes et chaque système bicyclique possédant 0 à 5 hétéroatomes choisis parmi N, O et S, à condition que ces hétéroatomes ne soient pas des atomes d'oxygène et/ou de soufre vicinaux, les substituants qui sont décrits par R₁₁ étant situés à n'importe quelles positions du système cyclique ;
le terme "alcényle" désigne un hydrocarbure aliphatique insaturé qui peut être à chaîne droite ou ramifiée, ou cyclique, et contient 2 à 12 atomes de carbone et 1 à 6 doubles liaisons ;
le terme "alkylacyle" désigne un groupe alkyl-C(O)- ; et
le terme "uréido" désigne un groupe R₆R₇N-C(O)-NR₆ où R₆ et R₇ sont tels que définis ci-dessus.

2. Composé selon la revendication 1, dans lequel R₅ et R₆ sont des groupes aryle, le terme "aryle" étant tel que défini dans la revendication 1.

3. Composé selon la revendication 1, choisi dans la classe formée par :
la diphénylphosphinyloxyméthylcétone d'acide N-[4-(N,N-diméthylaminométhyl)]benzoyl-L-valyl-L-aspartique,
la diphénylphosphinyloxyméthylcétone d'acide N-benzyloxycarbonyl-L-valyl-L-aspartique,
la diphénylphosphinyloxyméthylcétone d'acide N-benzyloxycarbonyl-L-aspartique,
la (*p*-chlorophényl)phénylphosphinyloxyméthylcétone d'acide N-benzyloxycarbonyl-L-aspartique et
la *(p*-méthoxyphényl)phénylphosphinyloxyméthylcétone d'acide N-benzyloxycarbonyl-L-aspartique.

4. Composé selon la revendication 1, choisi dans la classe formée par :
la diphénylphosphinyloxyméthylcétone d'acide N-benzyloxycarbonyl-L-valyl-L-alanyl-L-aspartique,
la diphénylphosphinyloxyméthylcétone d'acide N-[4-(N,N-diméthylaminométhyl)]benzoyl-L-valyl-L-alanyl-L-aspartique,
la (*p*-méthoxyphényl)phénylphosphinyloxyméthylcétone d'acide N-benzyloxycarbonyl-L-valyl-L-aspartique,
la (*p*-chlorophényl)phénylphosphinyloxyméthylcétone d'acide N-benzyloxycarbonyl-L-valyl-L-aspartique et
la di(*p*-méthoxyphényl)phosphinyloxyméthylcétone d'acide N-benzyloxycarbonyl-L-aspartique.

5. Composé selon la revendication 1, choisi dans la classe formée par :
la (*m*-méthoxyphényl)phénylphosphinyloxyméthylcétone d'acide N-benzyloxycarbonyl-L-aspartique,
la diphénylphosphinyloxyméthylcétone d'acide N-4-(pyridyl)carbométhoxy-L-valyl-L-alanyl-L-aspartique,
la diphénylphosphinyloxyméthylcétone d'acide N-benzyloxycarbonyl-L-valyl-D-aspartique, et
la diphénylphosphinyloxyméthylcétone d'acide N-3-(quinuclidinyl)carbonyl-L-valyl-L-alanyl-L-aspartique.

6. Composé selon la revendication 1, choisi dans la classe formée par :
la diméthylphosphinyloxyméthylcétone d'acide N-benzyloxycarbonyl-L-valyl-L-aspartique,
la (méthyl)(4-(2-méthylpropyl)phényl)phosphinyloxyméthylcétone d'acide N-benzyloxycarbonyl-L-valyl-L-aspartique,
la (phényl)((4-phényl)phényl)phosphinyloxyméthylcétone d'acide N-benzyloxycarbonyl-L-valyl-L-aspartique,
la phénylphosphinyloxyméthylcétone d'acide N-benzyloxycarbonyl-L-valyl-L-aspartique et
la (méthyl)((4-phényl)phényl)phosphinyloxyméthylcétone d'acide N-benzyloxycarbonyl-L-valyl-L-aspartique.

7. Composition pharmaceutique destinée à inhiber l'interleukine-1β)-protéase, comprenant un composé de formule (I) ou un sel pharmaceutiquement acceptable de celui-ci selon l'une quelconque des revendications précédentes, dans un excipient pharmaceutiquement acceptable.

8. Utilisation d'un composé de formule (I) ou d'un sel pharmaceutiquement acceptable de celui-ci selon l'une quelconque des revendications 1 à 6, ou d'une composition pharmaceutique selon la revendication 7, pour la préparation d'un médicament destiné à inhiber l'activité d'interleukine-1β-protéase chez un mammifère ayant besoin d'un tel traitement.
